# EUROPEAN PATENT APPLICATION

(11) **EP 4 261 154 A1**
(43) Date of publication of application: **18.10.2023**
(21) Application number: 21902045.0
(22) Date of filing: 30.07.2021
(51) Int. Cl.: B65F 7/00, B65F 1/16

(54) **STERILIZATION AND DEODORIZATION WASTE CONTAINER HAVING DUAL-WAVE BAND ULTRAVIOLET LAMP TUBE**

(30) Priority: 12.12.2020 CN 202022965269 U
(71) Applicant: Fujian Nashida Electronic Incorporated Company, Fuzhou, Fujian 350401 (CN)
(72) Inventor: WANG, Shiping, Fuzhou, Fujian 350401 (CN); CHEN, Jiangqun, Fuzhou, Fujian 350401 (CN); LUO, Youxi, Fuzhou, Fujian 350401 (CN); LIN, Zhou, Fuzhou, Fujian 350401 (CN)
(74) Representative: Dall'Olio, Christian
(86) International application number: PCT/CN2021/109449
(87) International publication number: WO 2022/121335

(57) **Abstract**

The present utility model relates to a sterilization and deodorization garbage bin, in particular, a sterilization and deodorization garbage bin with a dual-band ultraviolet lamp tube,the structure being characterized by further comprising an isolated cavity mounted on an inner side surface of the bin cover and a dual-band ultraviolet lamp tube mounted in the isolated cavity, the dual-band ultraviolet lamp tube being a lamp tube capable of simultaneously generating a direct sterilizing ultraviolet light wave and an ozone sterilizing ultraviolet light wave, the isolated cavity including a reflective hood and a transparent quartz glass, an opening of the reflective hood facing an inner cavity of the bin body, and the shape and size of the transparent quartz glass being adapted to an opening surface of the reflective hood, the opening of the reflective hood being covered by a sealing silicone ring. The advantages are that it effectively improves the operating environment of the ultraviolet lamp tube and avoids affecting internal circuits and mechanical components outside the isolated cavity, thereby improving the service life and reducing the use cost,and solving the problem of light transmission and irradiation of the ultraviolet lamp tube. The double disinfection and sterilization of ultraviolet ray can sufficiently and effectively disinfect and sterilize the garbage bin, greatly improving the disinfection and sterilization effect.

## Description

### Technical Field

The present utility model relates to a sterilization and deodorization garbage bin, in particular, a sterilization and deodorization garbage bin with a dual-band ultraviolet lamp tube. The utility model is applicable to a garbage bin with a cover.

### Description of Related Art

The existing domestic garbage bins are generally provided with a cover, and the cover is opened by means of foot stepping, sensing and light touch. Garbage bins, especially for use in kitchens, are prone to the growth of bacteria and the emission of odors, causing pollution to the environment in which people live, further affecting their smell and health, with health risks. In order to solve the above-mentioned problems, the prior art adopts ultraviolet light wave sterilization, namely, installing a direct ultraviolet lamp tube in a garbage bin to sterilize the environment inside the bin. However, this solution adopting ultraviolet direct sterilization has the following disadvantages:
1, The ultraviolet rays irradiated by the ultraviolet lamp tube of direct sterilization can only travel along a straight line, and there are more positions that cannot be irradiated when the inside of the bin is sterilized, and there is a dead angle in sterilizing, so that the sterilizing effect is also poor.
2, In order to ensure the light transmission and sterilization effect, the ultraviolet lamp tube in the prior art usually adopts a naked installation method, i.e. the ultraviolet lamp tube faces into the bin without shielding; due to the poor environment in the barrel, the operating conditions of the ultraviolet lamp tube are directly affected, so that the service life is also greatly shortened, the use cost is high, and it is difficult to promote.

### Brief Summary of the Invention

The purpose of the utility model is to provide a sterilization and deodorization garbage bin with a dual-band ultraviolet lamp tube capable of avoiding a disinfection angle, improving a disinfection effect, improving the operating environment of the ultraviolet lamp tube, improving the service life thereof and reducing the use cost according to the deficiencies of the prior art.

The object of the utility model is achieved by the following means:
A sterilization and deodorization garbage bin with a dual-band ultraviolet lamp tube, comprising a bin body, a bin cover and a control circuit,the structure being characterized by further comprising an isolated cavity mounted on an inner side surface of the bin cover and a dual-band ultraviolet lamp tube mounted in the isolated cavity, the dual-band ultraviolet lamp tube being a lamp tube capable of simultaneously generating a direct sterilizing ultraviolet light wave and an ozone sterilizing ultraviolet light wave, the isolated cavity including a reflective hood and a transparent quartz glass, an opening of the reflective hood facing an inner cavity of the bin body, and shape and size of the transparent quartz glass being adapted to an opening surface of the reflective hood, the transparent quartz glass being covered on the opening of the reflective hood via a sealing silicone ring, a control input end of the drive circuit of the dual-band ultraviolet lamp tube being connected to a drive control end of the control circuit via a control switch.

Thus, the main points of the utility model include:
1) By providing the isolated cavity for isolating the outside air, and sealing the dual-band ultraviolet lamp tube therein by means of the reflective hood and the transparent quartz glass connected in a sealed manner, only the glass surface needs to be wiped when it is dirty, so that the harsh environment in the garbage bin hardly affect the ultraviolet lamp tube in the isolated cavity, optimizing the operating environment of the ultraviolet lamp tube, improving the service life and reducing the use cost.
2) The transparent quartz glass can improve the transmittance of the ultraviolet light, and the reflective hood can reflect most other ultraviolet light waves in the isolated cavity to the transparent quartz glass and the ultraviolet light waves penetrate the glass to irradiate into the barrel, which greatly increases the intensity and quantity of effective light waves and solves the problem of ultraviolet light transmission and irradiation of the ultraviolet lamp tube.
3) The dual-band ultraviolet lamp tube provides direct sterilization and ozone sterilization for double disinfection. The direct sterilizing ultraviolet light wave has high photon energy, which can penetrate the cell membrane and cell nucleus of the microorganism, destroy the molecular bond of its DNA, and make it lose replication ability or lose activity and die. The ultraviolet light wave of ozone sterilization can change the O₂ (oxygen) in the air into O₃ (ozone). The ozone has a strong oxidation effect, and can diffuse in the bin and effectively disinfect bacteria. The dispersion property of ozone can just make up for the shortcoming that the direct sterilizing ultraviolet ray only travels along the straight line and there is a dead angle in sterilizing. The two kinds of sterilizing ultraviolet rays can sufficiently and effectively disinfect and sterilize the garbage bin, greatly improving the sterilization effect. In addition, in the process of disinfection and oxidation, the excess ozone is combined into oxygen within 30min, so that ozone contamination can be avoided.
4) The setting of the isolated cavity provides a good sterilization environmental basis for ozone sterilization of ultraviolet light waves. Since ozone is highly corrosive, it can only diffuse in the isolated cavity and in the interior cavity of the bin body without affecting the electrical circuit components and mechanical components of the interior space of the bin cover by means of the arrangement of the isolated cavity.

The utility model can further specifically be provided as follows:
The reflective hood is either a plastic plated part, or a resin hood with a reflective film on an inner side, or a stainless steel metal part with a reflective arrangement on an inner side.

The reflective hood is made of a material which is not easy to be aged and is not easy to be corroded and oxidized, so as to avoid being corroded by generated ozone and improve the reflection effect on light waves.

The direct sterilizing ultraviolet light wave is an ultraviolet light wave having a wavelength of 240-280 nm, preferably a direct sterilizing ultraviolet light wave having a wavelength of 254 nm. The direct sterilizing ultraviolet light wave is also called short-wave sterilizing ultraviolet (UVC). The ultraviolet light with a wavelength of 253.7-254nm has the strongest photon energy and the best disinfection effect.

The ozone sterilizing ultraviolet light wave is an ultraviolet light wave having a wavelength of 165-200 nm, preferably an zone sterilizing ultraviolet light wave having a wavelength of 185 nm. The ozone sterilizing ultraviolet light wave (a part of light wave of UVD) is irradiated in the air to change O₂ (oxygen) in the air into O₃ (ozone), and the ozone sterilizing ultraviolet light wave of 185nm is a wave band of preferable effect.

The sealing silicone ring is a sealing ring having side-inverted U-shaped cross-section, and an edge of the transparent quartz glass is located in an inner cavity of the U-shaped structure, whereby the edge of the transparent quartz glass is completely enclosed in the sealing silicone ring.

The isolated cavity mounted at a lower side of the bin cover may be vibrated due to frequent up-and-down flipping movements of the bin cover, the transparent quartz glass is easily broken due to the vibration, and the structure with the fully clad edge can reduce the vibration damage of the transparent quartz glass and ensure the reliability of the structure. In addition, the silicone can prevent aging caused by ultraviolet irradiation.

The control switch is either a Hall sensor, an angle sensor or a limit switch, and includes a separable control driving element and a measurement and control element, wherein the control driving element is respectively connected to the dual-band ultraviolet lamp tube and the control circuit.

For example, the control driving element of the angle sensor can be mounted on a main rotating shaft of the bin cover. When the bin cover (or a cover plate, wherein the bin cover is composed of a cover plate and an annular shell) is opened at a certain angle (namely, when the bin cover or the cover plate is opened), the control circuit cuts off power supply to the ultraviolet lamp tube by receiving a signal from the angle sensor, so that it is extinguished; and when the bin cover (or the cover plate) is closed to a set angle (when the bin cover or the cover plate is closed), the ultraviolet lamp tube is controlled to be opened for disinfection. For example, the Hall sensor includes a Hall element as the control driving element and a magnet as the measurement and control element. When the magnet leaves a measurement range of the Hall element (namely, when the bin cover or the cover plate is opened), the ultraviolet lamp tube is turned off; and when the magnet enters the measurement range of the Hall element (namely, when the bin cover or the cover plate is closed), the ultraviolet lamp tube is turned on. Thus, when the bin cover or cover plate is opened, the control switch sends a signal, and the control circuit controls the dual-band ultraviolet lamp tube to be extinguished so as to prevent ultraviolet rays from damaging skin and eyes of human, and when the bin cover or cover plate is closed, the control switch sends a signal so that the control circuit opens the ultraviolet lamp tube in time, and disinfects the inside of the bin in time.

One side of the bin cover is hinged to the bin body, one side of the bin cover of the hinged position is provided with a box in which the control circuit is placed, a groove is provided in the box, and the isolated cavity is mounted in the groove; the control driving element and the measurement and control element of the control switch are respectively mounted on the bin cover and the bin body. When the bin cover and the bin body are in a non-detachable connection configuration, the isolated cavity will flip up and down with the bin cover, so that the control switch is preferably mounted at a side edge or an opening edge of the bin cover.

The bin cover comprises an annular shell and a cover plate, and a side edge of the cover plate is hinged to the annular shell; the annular shell is sleeved on the bin body, a control circuit is mounted in an inner cavity thereof, and the isolated cavity is mounted in a groove provided in the annular shell; the control switch is provided in two sets, where the control driving element and the measurement and control element of one set are respectively mounted on the annular shell and the bin body, and the control driving element and the measurement and control element of the other set are respectively mounted on the cover plate and the annular shell.

In normal use, the control switch can be triggered by the cover plate turning up and down, and when it is necessary to detach the annular shell from the bin body, the control switch can also be triggered, so as to effectively avoid the damage to human body caused by ultraviolet leakage.

In summary, the present utility model provides a sterilization and deodorization garbage bin with a dual-band ultraviolet lamp tube, wherein the dual-band ultraviolet lamp tube is mounted using an isolated cavity with a reflective hood and transparent quartz glass, which effectively improves the operating environment of the ultraviolet lamp tube and avoids affecting internal circuits and mechanical components outside the isolated cavity, thereby improving the service life and reducing the use cost,and solving the problem of light transmission and irradiation of the ultraviolet lamp tube. The double disinfection and sterilization of ultraviolet ray can sufficiently and effectively disinfect and sterilize the garbage bin, greatly improving the disinfection and sterilization effect.

### Brief Description of the Drawings

Fig. 1 is a schematic structural diagram of a sterilization and deodorization garbage bin with a dual-band ultraviolet lamp tube according to embodiment 1 of the present utility model.
Fig. 2 is an exploded schematic structural diagram of the sterilization and deodorization garbage bin shown in fig. 1;
Fig. 3 is a schematic structural diagram of an isolated cavity in the sterilization and deodorization garbage bin with a dual-band ultraviolet lamp tube;
Fig. 4 is an exploded schematic structural diagram of the isolated cavity;
Fig. 5 is a sectional schematic structural diagram of the sterilization and deodorization garbage bin with a dual-band ultraviolet lamp tube according to embodiment 1 of the present utility model;
Fig. 6 is a schematic structural diagram of a sterilization and deodorization garbage bin with a dual-band ultraviolet lamp tube according to embodiment 2 of the present utility model;
Fig. 7 is an exploded schematic structural diagram of the sterilization and deodorization garbage bin shown in fig.6;
Fig. 8 is a sectional schematic structural diagram of a bin cover portion of the sterilization and deodorization garbage bin shown in fig.6;

### Reference numerals:

1-bin cover, 11-annular shell, 111-annular bin head, 112-annular middle body seat, 12-cover plate, 13-control circuit, 131-Hall element, 132-magnet, 133-Hall element, 134-magnet, 14-on-off cover driving device, 2-bin body, 3-isolated cavity, 31-reflective hood, 32-transparent quartz glass, 321-sealing silicone ring, 33-dual-band ultraviolet lamp tube, 34-ultraviolet lamp drive circuit.

The utility model is further illustrated in combination with the following specific embodiments.

### Detailed Description of the Invention

### Preferred Embodiment 1

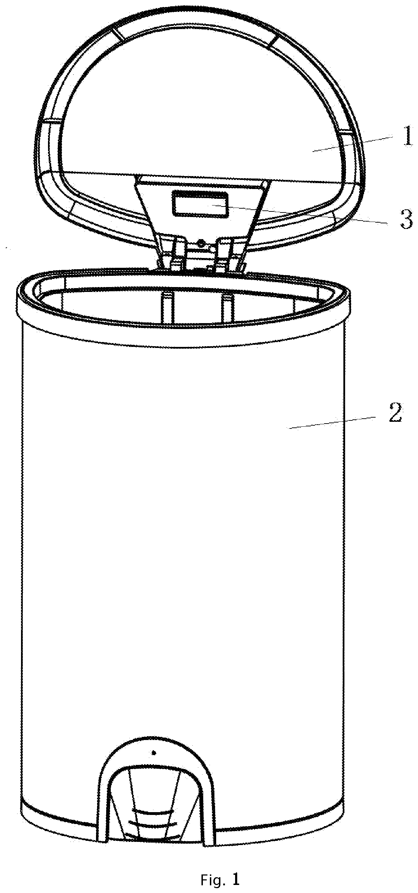

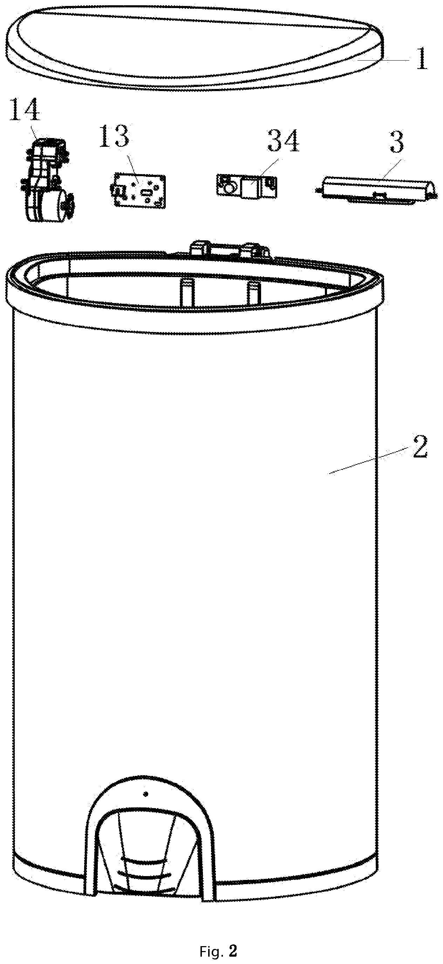

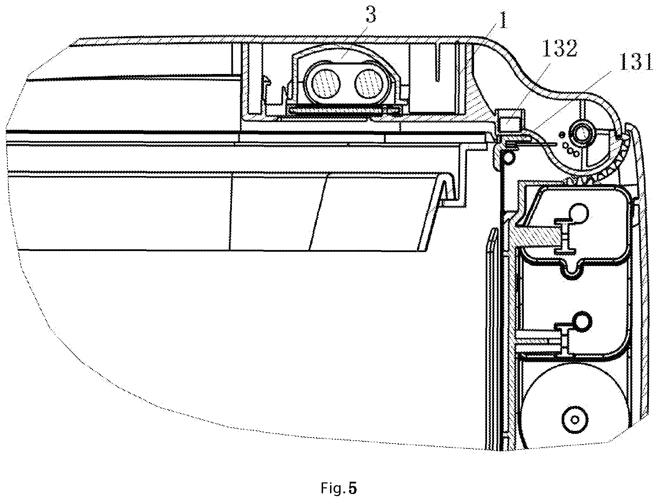

This embodiment is a sterilization and deodorization garbage bin with a dual-band ultraviolet lamp tube, and when the bin cover of the garbage bin cannot end away from the bin body (such as stepping on the garbage bin with a foot, touching the garbage bin with a foot, etc. and this embodiment refers to touching the garbage bin with a foot), the foot-touching control part, the opening and closing part of the bin cover, etc. belong to disclosed technology, and are not described in embodiment 1. With reference to figs. 1-5,
a sterilization and deodorization garbage bin with a dual-band ultraviolet lamp tube includes a bin cover 1, a bin body 2 for storing waste, and a control circuit 13. The bin cover 1 is hinged at an opening on an upper part of the bin body 2, and a control circuit 13 and an on-off cover driving device 14 are mounted on the bin body 2. An isolated cavity 3 isolated from air is provided in a lower part of the bin cover 1, and a light-transmitting window is provided below the isolated cavity 3. Since the environment of the garbage bin is poor, a dual-band ultraviolet lamp tube 33 with U-shaped structure is placed in one isolated cavity 3 isolated from air, and the penetrating ability of the ultraviolet light is very weak, and a transparent quartz glass 32 with a very high light transmittance is required to be used as the light-transmitting window of the isolated cavity 3. Ultraviolet light with a wavelength of 254 nm and a wavelength of 185 nm is generated by the dual-band ultraviolet lamp tube 33, wherein the ultraviolet light irradiates into the garbage bin through the window constituted by the quartz glass 32, and the ultraviolet light is used to perform sterilization and deodorization, wherein the wavelength of 254 nm (also referred to as short-wave sterilized ultraviolet light) has a relatively high photon energy, and when the microorganism is irradiated, it can penetrate the cell membrane and cell nucleus of the microorganism, destroy the molecular bond of the DNA thereof, so that the microorganism loses replication capacity or loses activity and dies. However, when the wavelength of 185nm is irradiated in the air, O₂ (oxygen) in the air can be changed into O₃ (ozone). Ozone has a strong oxidation effect, and can effectively kill bacteria. The diffusivity of ozone can just make up for the shortcoming that the disinfection has a dead point due to the transmission of ultraviolet only along a straight line, and strengthen the function of sterilization and deodorization.

The isolated cavity 3 is composed of a reflective hood 31, a transparent quartz glass 32 and a sealing silica silicone ring 321, wherein a peripheral ring of the transparent quartz glass 32 is fixedly protected by the sealing silicone ring 321 with a "U"-shaped cross section, and the dual-band ultraviolet lamp tube 33 is placed in the closed isolated cavity 3 composed of the reflective hood 31 and the transparent quartz glass 32. Since ultraviolet rays have a strong aging ability and ozone has a strong corrosiveness, the dual-band ultraviolet lamp tube 33 is placed in the closed isolated cavity 3. On the one hand, the ultraviolet light waves are prevented from being irradiated on plastic structural parts of a driving control mechanism, but concentrated on the garbage position in the bin, and on the other hand, the ozone generated by irradiation of the ultraviolet light waves is prevented from being emitted into an inner space of the bin cover 1 which causes that the control circuit and internal structural components are corroded and oxidized by ozone, and the dual-band ultraviolet lamp tube 33 is isolated and protected from being contaminated. If the transparent window is soiled, it is only necessary to wipe the transparent quartz glass 32 to ensure that the ultraviolet light sufficiently irradiates. The reflective hood 31 (wherein the reflective hood 31 can be a material which is not easy to be aged and not easily corroded and oxidized, such as a plastic plated part, a reflective film or a stainless steel metal part, etc. and the reflective hood 31 in this embodiment refers to a plastic plated part) can reflect ultraviolet light waves into the garbage bin, greatly increasing the effective light wave intensity. The ultraviolet light waves are reflected by the reflective hood 31 and can only be irradiated into the garbage bin through the transparent quartz glass 32. The transparent quartz glass 32 can simultaneously transmit most ultraviolet light waves with a wavelength of 254 nm and a wavelength of 185 nm, so as to ensure that most ultraviolet rays can be irradiated into the garbage bin for sterilization and generate ozone for sterilization and deodorization in the garbage bin. Since the silicone has a strong aging resistance, the sealing silicone ring 321 used in the present embodiment is sealing silicone with a U-shaped cross section.

The bin cover 1 cannot end away from the bin body 2. A control switch is mounted on the bin body 2 (the control switch can be a Hall element, a limit switch, an angle sensor, etc. and the present embodiment uses a Hall element, namely, a Hall element 131). A magnet 132 is mounted on the bin cover 1. When the bin cover 1 is opened, the magnet 132 leaves a measurement range of the Hall element 131 along with the bin cover 1, and after receiving a signal from the Hall element 131, the control circuit 13 controls the dual-band ultraviolet lamp tube 33 to be extinguished via an ultraviolet lamp drive circuit 34 so as not to generate ultraviolet rays, so as to prevent ultraviolet rays from damaging skin and eyes of human. When the bin cover 1 is closed in place, the Hall element 131 detects that the magnet 132 is close, and the control circuit 13 controls the dual-band ultraviolet lamp tube 33 to be opened; otherwise, the dual-band ultraviolet lamp tube 33 is always in an extinguished state.

The parts not described in this embodiment are the same as those in the prior art.

### Preferred Embodiment 2

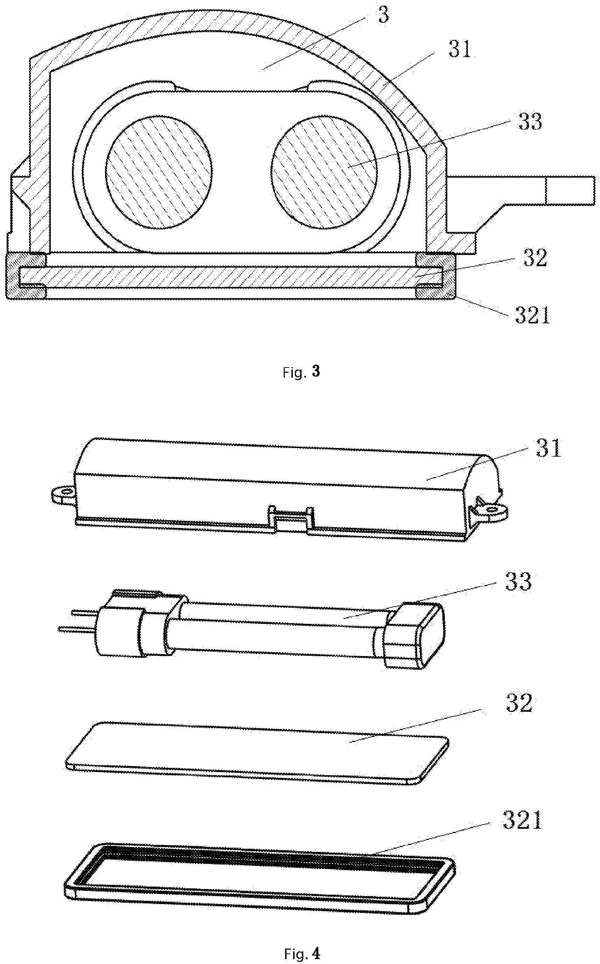

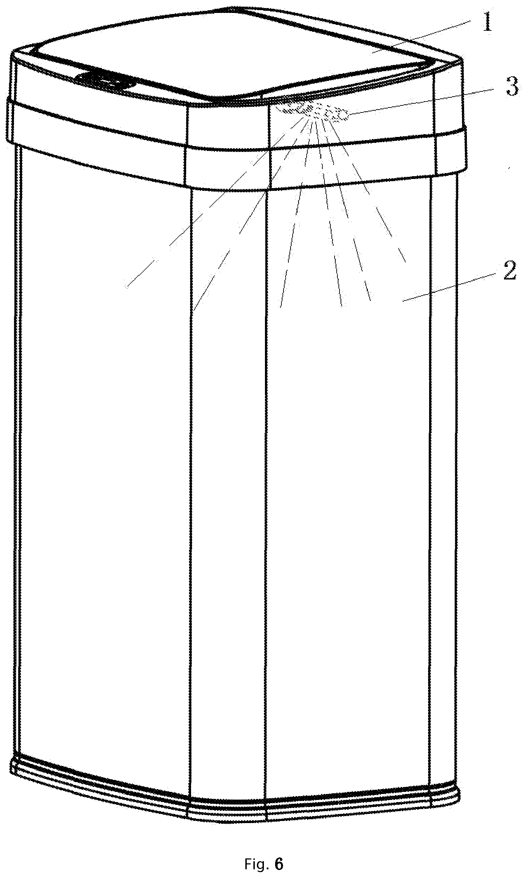

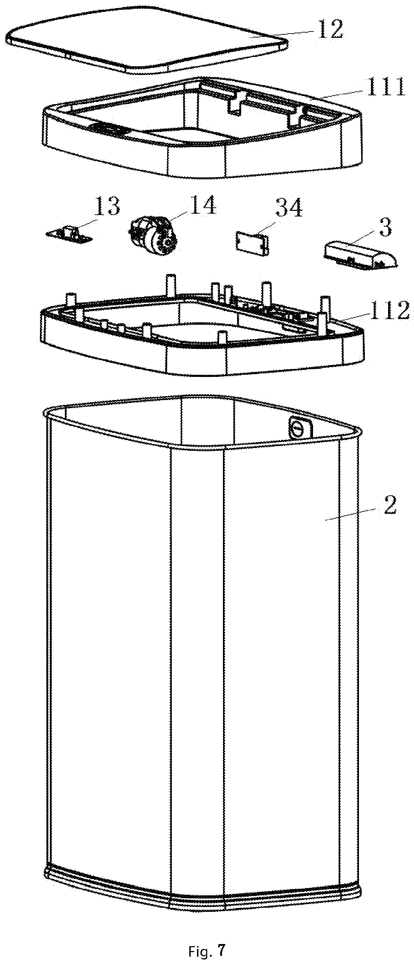

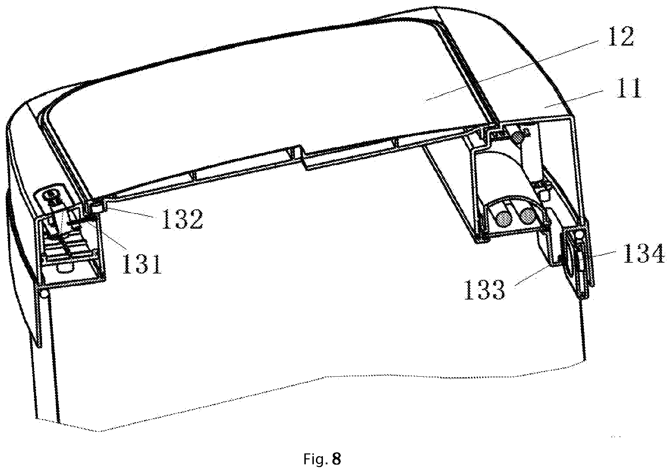

This embodiment is a sterilization and deodorization induced garbage bin with a dual-band ultraviolet lamp tube, and when the bin cover of the garbage bin can end away from the bin body (such as inducing the garbage bin, etc. and this embodiment refers to inducing the garbage bin), the infrared induced part, the opening and closing part of the bin cover, etc. belong to disclosed technology, and are not described in the embodiment. With reference to figures 3, 4 and 6-8, a sterilization and deodorization garbage bin with a dual-band ultraviolet lamp tube includes a bin cover 1, a bin body 2 for storing waste, and a control circuit 13. The bin cover 1 includes an annular shell 11 (the annular shell 11 is composed of 111- annular bin head and an annular middle body seat 122) and a cover plate 12, the cover plate 12 is hinged on the annular shell 11, and the bin cover 1 is placed at an opening on an upper part of the bin body 2. A control circuit 13 and an on-off cover driving device 14 are mounted in a cavity of the annular shell 11 formed after the annular bin head 111 and the annular middle body seat 112 are fastened together. An isolated cavity 3 placed in isolation from air is also mounted below the bin cover 1. A dual-band ultraviolet lamp tube 33 is arranged in the isolated cavity 3. A light-transmitting window composed of a transparent quartz glass 32 with an extremely high light transmittance is arranged below the isolated cavity 3. Ultraviolet light with a wavelength of 254 nm and a wavelength of 185 nm generated by the dual-band ultraviolet lamp tube 33 irradiates into the garbage bin through the window composed of the quartz glass 32. The ultraviolet light waves are used for sterilization and deodorization, wherein ultraviolet light waves with a wavelength of 185 nm can generate ozone for sterilization and deodorization, and the use of the diffusivity of ozone can enable the odor to fill the whole space of the garbage bin, which can just make up for the shortcoming that ultraviolet rays only travel along a straight line and there is an dead angle in sterilizing, enhancing the function of sterilization and deodorization.

The structure, material selection and function of the isolated cavity 3, the reflective hood 31, the transparent quartz glass 32, the sealing silicone ring 321 and the dual-band ultraviolet lamp tube 33, and the sterilization and deodorization principle of the dual-band ultraviolet lamp tube 33 can be described with reference to the description in embodiment 1, and are not described in this embodiment.

The bin cover 1 can end away from the bin body 2, the bin cover 1 is composed of the annular shell 11 and the cover plate 12, a control switch is mounted on the bin cover 1 (the control switch can be a Hall element, a limit switch, an angle sensor, etc. and the present embodiment uses a Hall element, namely, a Hall element 131 and a Hall element 133). A magnet 134 is mounted on the bin body 2. When the bin cover 1 ends away from the bin body 2, the magnet 134 leaves a measurement range of the Hall element 133, and after a signal is generated by the Hall element 133, the control circuit 13 controls the dual-band ultraviolet lamp tube 33 to extinguish and no longer generate ultraviolet rays, so as to prevent ultraviolet rays from damaging skin and eyes of human. Similarly, a magnet 132 is mounted on the cover plate 12, and when the cover plate 12 is opened, the magnet 132 moves away from a measurement range of the Hall element 131 along with the cover plate 12, and after the Hall element 131 generates a signal, the control circuit 13 controls the dual-band ultraviolet lamp tube 33 to extinguish and no longer generate ultraviolet rays, so as to prevent ultraviolet rays from damaging human skin and eyes. When the cover plate 12 is closed in place and the bin cover 1 is placed on the bin body 2, the control circuit 13 controls the dual-band ultraviolet lamp tube 33 to be turned on for operation only when these two conditions are satisfied simultaneously, otherwise the dual-band ultraviolet lamp tube 33 is always in an off state. On the basis of the foregoing content, a killing time of the ultraviolet lamp tube can also be set.

The parts not described in this utility model are the same as those in the prior art.

The foregoing description is merely preferred embodiments of the present utility model, and is not intended to limit the present utility model. Any modifications, equivalents, improvements, etc. within the spirit and principles of the present utility model are intended to be included within the scope of the present utility model.

## Claims

1. A sterilization and deodorization garbage bin with a dual-band ultraviolet lamp tube, comprising a bin body, a bin cover and a control circuit,**characterized by** further comprising an isolated cavity mounted on an inner side surface of the bin cover and a dual-band ultraviolet lamp tube mounted in the isolated cavity, the dual-band ultraviolet lamp tube being a lamp tube capable of simultaneously generating a direct sterilizing ultraviolet light wave and an ozone sterilizing ultraviolet light wave, the isolated cavity including a reflective hood and a transparent quartz glass, an opening of the reflective hood facing an inner cavity of the bin body, and shape and size of the transparent quartz glass being adapted to an opening surface of the reflective hood, the transparent quartz glass being covered on the opening of the reflective hood via a sealing silicone ring, a control input end of the drive circuit of the dual-band ultraviolet lamp tube being connected to a drive control end of the control circuit via a control switch.

2. The sterilization and deodorization garbage bin with a dual-band ultraviolet lamp tube according to claim 1, **characterized in that** the reflective hood is either a plastic plated part, or a resin hood with a reflective film on an inner side, or a stainless steel metal part with a reflective arrangement on an inner side.

3. The sterilization and deodorization garbage bin with a dual-band ultraviolet lamp tube according to claim 1, **characterized in that** the direct sterilizing ultraviolet light wave is an ultraviolet light wave having a wavelength of 240-280 nm, preferably a direct sterilizing ultraviolet light wave having a wavelength of 254 nm.

4. The sterilization and deodorization garbage bin with a dual-band ultraviolet lamp tube according to claim 1, **characterized in that** the ozone sterilizing ultraviolet light wave is an ultraviolet light wave having a wavelength of 165-200 nm, preferably an zone sterilizing ultraviolet light wave having a wavelength of 185 nm.

5. The sterilization and deodorization garbage bin with a dual-band ultraviolet lamp tube according to claim 1, **characterized in that** the sealing silicone ring is a sealing ring having side-inverted U-shaped cross-section, and an edge of the transparent quartz glass is located in an inner cavity of the U-shaped structure, whereby the edge of the transparent quartz glass is completely enclosed in the sealing silicone ring.

6. The sterilization and deodorization garbage bin with a dual-band ultraviolet lamp tube according to claim 1, **characterized in that** the control switch is either a Hall sensor, an angle sensor or a limit switch, and includes a separable control driving element and a measurement and control element, wherein the control driving element is respectively connected to the control circuit.

7. The sterilization and deodorization garbage bin with a dual-band ultraviolet lamp tube according to claim 1, **characterized in that** one side of the bin cover is hinged to the bin body, one side of the bin cover of the hinged position is provided with a box in which the control circuit is placed, a groove is provided in the box, and the isolated cavity is mounted in the groove; the control driving element and the measurement and control element of the control switch are respectively mounted on the bin cover and the bin body.

8. The sterilization and deodorization garbage bin with a dual-band ultraviolet lamp tube according to claim 1, **characterized in that** the bin cover comprises an annular shell and a cover plate, and a side edge of the cover plate is hinged to the annular shell; the annular shell is sleeved on the bin body, a control circuit is mounted in an inner cavity thereof, and the isolated cavity is mounted in a groove provided in the annular shell; the control switch is provided in two sets, where the control driving element and the measurement and control element of one set are respectively mounted on the annular shell and the bin body, and the control driving element and the measurement and control element of the other set are respectively mounted on the cover plate and the annular shell.
